# EUROPEAN PATENT APPLICATION

(11) **EP 1 925 718 A2**
(43) Date of publication of application: **28.05.2008**
(21) Application number: 07120785.6
(22) Date of filing: 15.11.2007
(51) Int. Cl.: D06M 11/74, D06M 11/83, D06M 23/16, A61B 5/103, G01L 3/10, G01L 5/10

(54) **Process for making a textile comprising integrated conductive areas and textile thereby obtained**

(30) Priority: 21.11.2006 IT MI20062224
(71) Applicant: Manifatture Cotoniere Settentrionali S.R.L., 21052 Busto Arsizio (Varese) (IT); Microsystems S.r.l., 20124 Milano (IT)
(72) Inventor: Ceriotti, Francesco, 21052 BUSTO ARSIZIO (VARESE) (IT); Tausel, Marco, 20123 Milano (IT)
(74) Representative: Baroni, Matteo

(57) **Abstract**

A process for making a flexible substrate provided with one or more electrically conductive areas (6, 6a, 6b) comprising: providing a flexible substrate (1) made of a sheet material; providing forming means (4) for at least one first electrically conductive area, said means (4) having a profile (4a) delimiting an area defined on said substrate (1), and a thickness defined along a direction substantially orthogonal to said substrate (1); applying an electrically conductive paste onto said flexible substrate (1) by means of said forming means (4), thereby obtaining a first electrically conductive area (6, 6a) having the shape delimited by the area and the thickness defined by said profile (4a); causing solidification of the conductive paste of said first conductive area (6, 6a); causing mutual engagement between the conductive paste of said first conductive area (6 6a) and said substrate (1).

## Description

The present invention relates to a process for making a textile or flexible substrate comprising conductive areas, and to the related textile or substrate obtained by said process. In more detail, it relates to manufacture of conductive areas on a textile or flexible substrate of any nature, which areas are adapted to acquire or send electric signals or others; the invention also relates to manufacture, on a textile or flexible substrate of any nature, of conductive areas or conductors suitable for carrying said signal to any point of said textile. The invention further relates to the process for making said textile comprising said conductive areas and said conductors or other conductors in contact with said conductive areas, and to the related textile obtained by said process.

The application field of the present invention is that of conductive textiles or textiles provided with sensors for producing garments with sensors incorporated thereinto or capable of transmitting or receiving signals of the electric or electronic type, and useful for monitoring body signals.

The electric signals generated by a body, such as the signal of an electrocardiogram, are of small intensity and require that an optimal contact exists between the skin and the electrode, as well as an optimal electric conduction between the electrode and the device receiving, transmitting or recording the signal. A long-duration monitoring operation must be done in a comfortable manner, by means of a garment having conductive areas used as electrodes and/or sensors, for example.

Conductive textiles also have other applications.

Several different industrial sectors need materials made of flexible substrates or textiles, having conductive areas connected, by suitable conductors, to other areas of the same material or to measurement or transmission devices. These areas, also made of several layers, can be used as sensors too, for detecting several environmental or body properties, making electric circuits, building resistors for heat production.

Known in the art are conductive textiles obtained through use of conductive fibres. In particular, for making conductive areas on a textile, two techniques are used: during the weaving step a thread consisting of a conductive fibre is inserted, or a thread consisting of a conductive fibre is embroidered on the previously existing textile. Both the above processes have many problems.

When these conductive textiles are obtained through weaving, the above mentioned problems are mainly represented by high working costs, difficulties in weaving a conductive fibre, and above all difficulties in positioning the conductive fibre close to predetermined areas, in the desired sizes and in a shape different from the rectangular one; therefore drawing a circuit becomes difficult and expensive.

If the embroidery technique with a conductive fibre is used, positioning is easier, and any shape can be given to the conductive area, but problems arise in terms of difficulty in manufacturing this area and high costs.

In addition to the difficulties present in the two above described techniques, there is in any case the problem of an unsatisfactory electric contact, because said contact is mainly obtained at points where the conductive fibre projects externally of the textile, so that a continuous conductive surface cannot be obtained.

Other methods of making conductive textiles are known. In US5723186 and EP1284278A2 processes for making conductive textiles are disclosed in which a flexible non conductive substrate is wetted with an aqueous solution containing a conductive material and glue, and the solution is then dried making said glue active.

However these processes cannot be used for making either shaped areas or areas or regions with different electric properties; in addition, it is not possible to obtain accurate geometries, and connections to external devices or other conductors or conductive areas is not provided by this technique. Furthermore, thickness of the conductive region is given by the concentration of the aqueous solution and by the impregnated quantity. Thickness is therefore a difficult and non independent control property.

A silk-screen process for printing circuits with conductive ink on a flexible substrate is disclosed in US2002/0047107. The resulting conductive region is thin, and has no possibility of forming a connection in a simple manner with the conductors extended from one side to the other of the textile or made of a material different from the ink therein used.

For practical uses it is important to be able to connect the conductive areas with each other or with external devices. The known art uses connectors to be fixed to the textile as in US2006128169 or in US5371326. Use of a connector increases the number of procedures to be performed for reaching the desired operation and produces an uneven surface at the contact point, that can give rise to problems in applications where surface smoothness is required, as in the case of measurement of body parameters where the textile is in contact with the skin.

A similar situation appears in US2002/0076949 where connection is obtained by sewing other textiles onto the concerned garment. Other solutions, disclosed in US2006/0027552A1 for example, contemplate welding or gluing of the conductor to the conductive region, which procedure is complicated and again gives an uneven contact surface.

The present invention discloses a process for making a textile or a flexible substrate containing conductive regions, and the related textile or substrate obtained by said process.

The invention in particular solves the above problems and allows acquisition, transmission and possible variation of electric signals or other types of signals, as well as manufacture of conductive paths or circuits on a textile or flexible substrate for carrying an electric signal to any textile or substrate point, or to an external device.

It is an object of the invention to provide a process for producing conductive regions in a convenient and cheap manner on predetermined positions of a textile or flexible substrate, which regions can be electrically connected with each other or with external devices.

It is another object of the invention to provide a process for producing conductive regions of a controllable thickness on a textile or flexible substrate, to be used for particular electric, metric and physiological purposes.

It is another object of the invention to provide a process for producing conductive regions of predetermined thickness with a selected shape and surface topography, to be made on a textile or a flexible substrate.

It is another object of the invention to provide a process for producing conductive regions on a textile which are directly connected to conductive paths, conductors or circuits used for carrying signals or electric energy and maintaining these mechanical and/or electric connections protected.

It is another object of the invention to provide a process for obtaining a shaped conductive region and conductive paths placed on either side of a textile or a substrate, said paths being adapted to provide a sure and efficient mechanical and/or electric connection between the different electric or electronic components placed on the textile or flexible substrate or on external devices.

It is another object of the invention to provide a process for obtaining shaped conductive regions and conductive paths capable of minimising the influence of the variations in the moisture degree of the textile on the desired functions.

It is another object of the invention to make available a process for producing a textile from which a garment can be made that is adapted to be used for monitoring parameters of the human or animal body.

It is another object of the invention to make available a method and a textile for manufacturing garments, making monitoring of body parameters less restrictive, uncomfortable or difficult.

It is another object of the invention to make available a method and a textile for manufacturing a device adapted to monitor the activity of a human or animal body that has a simple structure and low manufacturing costs.

The foregoing and other objects are substantially achieved by a process for making a textile or a flexible substrate, hereinafter jointly called "textile", containing conductive regions, according to the features described in the appended claims.

It is a further object of the present invention to provide a textile obtained by the above described process. This textile can be used for producing garments, adapted to read measurements on the human body in the medical and clinical field, to electrically stimulate muscles, to build sensors for detecting variations in the environmental parameters, to produce printed circuits.

Objects and advantages of the present invention will be apparent from the following detailed description of an embodiment of same (and variants thereof) and from the accompanying drawings given by way of non-limiting example, in which:
- Fig. 1 diagrammatically shows a flexible substrate in a step of the process in accordance with the invention;
- Fig. 2 shows a die adapted to form a conductive region having a circular design;
- Fig. 3 shows the substrate seen in Fig. 1 in a subsequent step of the process in accordance with the invention;
- Fig. 4 shows the substrate seen in Fig. 1 in another example of the process;
- Fig. 5 diagrammatically shows a cross-section view of the substrate seen in Fig. 1 in a further example of the process;
- Fig. 6 diagrammatically shows a substrate made in accordance with the invention where a multi-layer conductive area is obtained;
- Fig. 7 shows a substrate made in accordance with the invention in which the conductive area is designed to be connected to other devices through a conductive path.

The process being the object of the invention enables a flexible substrate 1 to be obtained which is provided with electric conduction areas 6, 6a, 6b formed on the original textile, that give rise to an electric conduction also between the two sides of the textile 1 and towards the outside from both sides or only one, as well as conductive paths applied to the textile surface and joining the conductive areas and/or enabling electric connection to the outside.

For making the conductive areas 6, 6a, 62 and the conductive paths 9, a suitable conductive paste is used which preferably consists of a resin enriched with a conductive material which can be liquid or solid, in powder or fibres. The resins used can be the normal resins already known in the field for printing textiles or flexible substrates. They include acrylic, phenolic, epoxy, polyurethane, polyester, cellulose, plastic, silicone, vinyl resins, etc. for example. The conductive material to be added to the selected resin can in turn be any type of liquid or solid, powdered or in fibre conductive material, adapted to be mixed with the above resins.

Examples of conductive materials that can be used are: carbon, precious and non precious metals, conductive polymers, etc.

The conductive material is mixed with the resin in percentages that can widely vary depending on the desired conductivity and/or resistance effect.

The support on which the conductive paste is applied, i.e. said flexible substrate 1, can be any type of textile, such as a knitted fabric, shuttle-made fabric, nonwoven fabric or other type of support commonly used in the textile field which can be elastic or not and made up of any natural, synthetic, artificial or other fibre; it can also be a suitable substrate made of paper, plastic, etc.

The process according to the present invention first of all comprises a step of providing a flexible substrate 1, preferably of the above type; the flexible substrate is made up of a sheet material, i.e. has a dimension - typically identified as thickness - much smaller than the two other dimensions.

Also provided are forming means 4 for a first electrically conductive area; the forming means 4 has a profile 4a delimiting a determined area on substrate 1, and a thickness determined along a direction substantially orthogonal to the substrate 1 itself. The perpendicular line used for determining said thickness is identified at said area defined on substrate 1, on which the first conductive area 6, 6a is formed.

The forming means 4 may comprises pressing means for example, that preferably consists of a die built with one or more suitably shaped rigid plates.

Through the forming means 4 a conductive paste preferably of the above stated type, is applied onto the flexible substrate 1 so as to obtain a first conductive area 6.

In particular, the first conductive area 6 has a shape defined by the profile 4a of the forming means 4; in more detail, by means of profile 4a the shape of the conductive area is delimited both as regards the projection of said conductive area onto substrate 1, and as regards the thickness of the first conductive area 6 defined along a direction orthogonal to the substrate 1 itself.

Therefore, through the forming means 4, thickness of the first conductive area 6 can be controlled.

For instance, profile 4a can be the inner profile of a through cavity of the forming means 4, into which the conductive paste is poured when it is not yet solidified.

The conductive paste of the first area 6 is then caused to solidify and engagement between this conductive paste of the first area 6 and the flexible substrate 1 is obtained.

Preferably the last-mentioned two steps are performed substantially simultaneously.

In particular, the solidification step and/or the step for obtaining mutual engagement between the conductive paste and substrate 1 are carried out by polymerisation of the conductive paste itself.

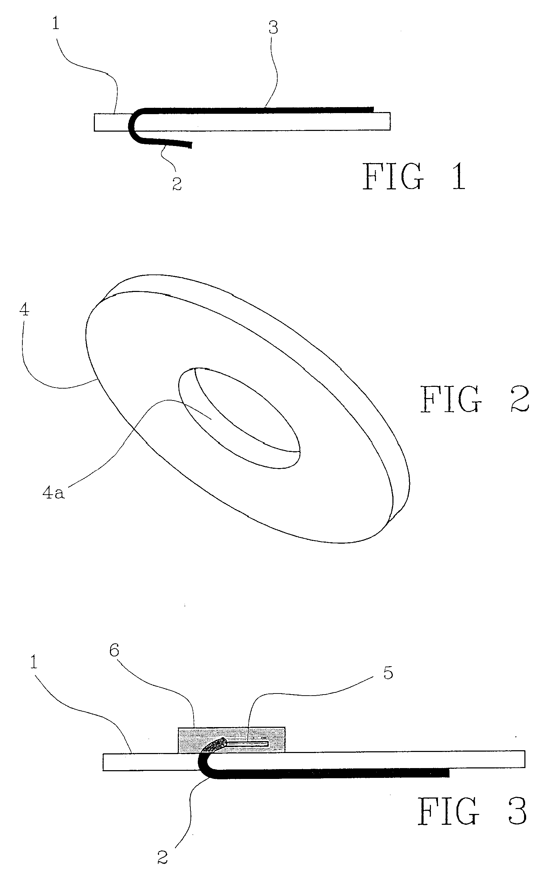

By way of example, the flexible substrate 1 (a textile for example, as above mentioned) is placed on an apparatus for silk-screen printing. The die 4, with the selected shape defining the design of the first conductive area 6 (a circle or an ellipse for example, as diagrammatically shown in Fig. 2), is such placed as to be able to print the conductive region in a predetermined part of textile 1.

The die 4 used is obtained by means of a stiff plate of a specified thickness and made of a material adapted to be shaped, by working it with a numeric-control machine for example, or using other methods known in the art, and with the accuracy allowed by the technique used in manufacturing this die.

The thickness of the layer thus laid down can be controlled by the thickness of the selected stiff plate for manufacturing die 4.

Die 4 is mounted in the apparatus for silk-screen printing in place of the usual frame for silk-screen printing, or is disposed directly over the textile 1 if a silk-screen printing apparatus is not used, but another known method for fixing the textile position on a printing plane is adopted.

Once the textile 1 and die 4 have been positioned, the conductive paste can be spread on the textile in the region defined by the die shape.

The process according to the present invention also enables printing with very viscous pastes, because the printing process can be assisted by application of pressure promoting covering and penetration of the textile by the paste.

In fact, provision is made for a step of providing auxiliary forming means (not shown) the shape of which matches that of said forming means 4 and a step of applying a predetermined pressure onto the conductive paste through said auxiliary forming means.

Practically, the forming means 4 have a through cavity provided with the above mentioned profile defining the design of the first conductive area 6; the auxiliary forming means have a raised design (high relief) with the same profile.

By moving the forming means 4 and the corresponding auxiliary forming means close to each other when the forming means 4 is still in contact with the flexible substrate 1 and in such a manner that the high relief of the auxiliary forming means is in register with the conductive paste laid down, it is possible to exert a predetermined pressure on the conductive paste, so as to promote adhesion between the conductive paste and substrate 1.

This pressure can be for example applied both by means of a second plate conforming in shape to the shape of the first plate (the latter defining the forming means 4), and by means of a roller made of elastic material, or by adopting another suitable method.

By adopting a second plate having a selected surface topography it is possible to give the conductive region the desired surface topography.

Laying of the conductive paste on substrate 1 is preferably carried out using a cold procedure and then preferably substrate 1 (together with the mentioned conductive paste) is submitted to a heating process (it is inserted into an oven, for example) for polymerisation of the bonding agent of the paste that becomes therefore fixed to the textile.

In this manner a greater resistance to external agents, such as those due to washing operations, is obtained as regards the printed area 6 of the conductive textile thus obtained.

The polymerisation process can also take place by a cold procedure, in a longer period of time.

Preferably, the process of the invention further comprises a step of providing an electrically non conductive layer 7 between the substrate 1 and the first conductive area 6.

In other words, the non conductive layer 7 can be made beforehand on substrate 1 in the region where the first conductive area 6 will be laid, so that, should the textile get wet, due to the presence of sweat for example, this does not cause an undesirable effect of electric conduction from the conductive area 6 to textile 1.

The electrically insulating layer 7 is laid by means of a die having a predetermined shape; preferably this shape is substantially coincident with the profile defined by the forming means 4 of the first conductive area 6 so that the electrically insulating layer 7 and the first conductive area 6 substantially have the same profile.

The process in accordance with the invention can also involve a step of engaging a conductive cable 3 with the substrate 1. In this case, the first conductive area 6 is preferably obtained at one end 2 of the conductive cable 3 and is in electric contact with this end.

In more detail, the conductive cable 3 has an electrically insulating sheath; said end 2 of cable 3 has a first portion 8 coated with the insulating sheath and a second portion 5 not covered by the insulating sheath.

Preferably, the non conductive layer 7 interposed between substrate 1 and first conductive area 6 incorporates the first portion 8 of the cable end 2, while the first conductive area 6 incorporates the second portion 5 of the end 2 of said cable 3.

By way of example, the conductive cable 3 can be sewn onto the flexible substrate 1 and be then exposed to the outside in an alternated manner on either side of the flexible substrate 1 itself.

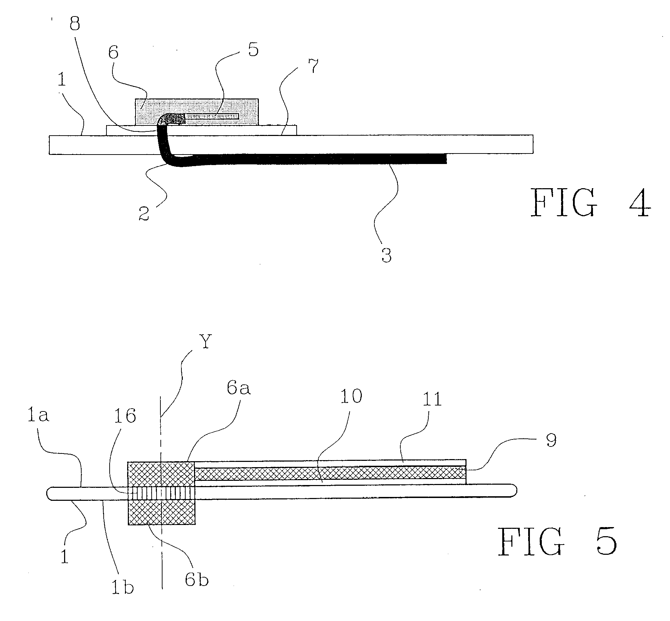

The process according to the invention can further comprise a step of making a second conductive area 6b on the opposite side of the flexible substrate 1 relative to the first conductive area; therefore, being denoted as the first surface 1a, the surface of the flexible substrate 1 on which the first conductive area is made (identified by 6a in Fig. 5), the second conductive area 6b is made on a second surface 1b of the flexible substrate 1.

Therefore forming means for the second conductive area 6b is provided, said means having a profile defining the desired shape for the second conductive area; in particular, the profile of the forming means of the second conductive area 6b delimits an area defined on substrate 1 and a thickness defined along a direction substantially orthogonal to the substrate 1 itself.

Preferably this profile is substantially coincident with the shape of the first conductive area. Then a conductive paste, preferably of one of the above described types, and in particular of the same type as that of the first conductive area 6a, is applied to the second surface 1b of the flexible substrate 1, through said forming means, so as to obtain the second conductive area 6b.

It is to be noted that the first and second conductive areas 6a, 6b at least partly overlap one another along an axis Y orthogonal to the flexible substrate 1.

The conductive paste of the second conductive area 6b is then solidified using one of the above described techniques for solidification of the conductive paste of the first area 6a.

Also obtained is a bond between the conductive paste of the second conductive area 6b and the flexible substrate 1.

Preferably, solidification of the paste of the first conductive area 6a and the paste of the second conductive area 6b takes place simultaneously, by a single polymerisation step for example.

Advantageously, for the conductive paste of the second conductive area 6b auxiliary forming means may be used, preferably of the same type as those described above for the first conductive area, in order to exert a predetermined pressure on this conductive paste and facilitate adhesion of same to substrate 1.

In a preferred embodiment, an insulating layer preferably substantially having the same profile as the second conductive area may be interposed between the paste of the second conductive area 6b and substrate 1.

It is to be noted that the electrically non conductive layer interposed between the first conductive area and the substrate, and/or the electrically non conductive layer interposed between the second conductive area and substrate 1 can be made of the same resin as used for the conductive paste, with the difference that no electrically conductive material is added to this resin, in the case of the insulating layer.

To enable a stronger mutual engagement between the two conductive areas 6a, 5b and keep the flexible substrate 1 steady between these conductive areas, a microperforation step is carried out on at least part of a predetermined region 16 of substrate 1, said predetermined region 16 being defined by the overlapped region between the first and second conductive areas 6a, 6b. This microperforation step is particularly advantageous in case of very "closed" or thick textiles or substrates or in any case textiles or substrates forming a barrier that can be hardly penetrated by the conductive paste.

By way of example, said microperforation can be obtained by any process adapted to perforate a textile (by mechanical punching, by laser, etc., for example), to promote penetration of the paste.

It is to be noted that the microperforation step can be also carried out when a conductive area on a single part alone of substrate 1 is made, i.e. without adding a second conductive area at least partly superposed over the first one, along a direction perpendicular to substrate 1; in this case microperforation is carried out close to the area on which the first conductive area is then made and has the task of improving adhesion between this first area (or the insulating layer laid down before the conductive paste of said first area) and substrate 1.

Obtaining of a conductive area or sensor through a microperforation process is diagrammatically shown in Fig. 5. The predetermined microperforation region 16 of substrate 1 is made where the conductive paste of the first area 6a is laid, with the aid of a shaped die defining the shape and position of the first area, and a second die complementary to the first one used for applying pressure (i.e. the above mentioned forming means 4 and auxiliary forming means) to enable the paste to penetrate into the holes and reach the other side of substrate 1.

After application of the paste of the first area 6a and possible polymerisation of same, a conductive paste is applied onto the other side of the textile, causing partial or full overlapping of the two parts along said axis Y orthogonal to the flexible substrate 1.

At all events, at least part of the printed area on one side of the textiles overlaps at least part of the printed area on the opposite side.

The two conductive areas 6a, 6b are in direct contact and amalgamated, and portions of electric contact between the two sides of the textile are obtained.

The process can also involve one or more steps of making other conductive areas on one or both surfaces of substrate 1.

Preferably, in order to mutually connect two conductive areas present on the same substrate surface, an electrically conductive path 9 can be made. The performed steps are substantially similar to those carried out for making a conductive area.

In fact, forming means (and, if necessary, auxiliary forming means for applying a suitable pressure) is provided, which means has a profile defining the path shape, and a conductive paste is applied onto the substrate so as to obtain a path 9 having this shape.

The conductive paste used can be of one of the above stated types for the conductive areas.

The conductive paste of the path is then solidified and engagement of same with substrate 1 is caused.

An electrically insulating layer 10 can be interposed between the conductive path 9 and flexible substrate 1.

The conductive path 9 can be made in order to carry the signal acquired by the sensors (i.e. by the conductive areas adapted to be brought into contact with the human or animal body), or to send a signal to the conductive areas from any point of the textile or from the outside.

In the preferred embodiment, an auxiliary insulating layer 11 is applied onto the first area and/or the second area and/or the conductive path on the opposite side (i.e. externally) relative to the flexible substrate 1, so as to electrically insulate these elements from the outside.

This insulating layer 11 can also cover possible other conductive areas or conductive paths made on the flexible substrate 1.

The above described laying process can be also used in order to obtain one or more multi-layer conductive areas 20.

The process through which multi-layer structures can be obtained is made by repeating the above described operation on the same region of substrate 1, the concerned substrate region being printed several times both with the same design or shape and with different designs. The multi-layer laying can be carried out on a single side of the textile or on both sides.

In fact, there is a step of providing at least one second conductive paste 20b that is laid on the conductive paste 20a of the first area and/or on the conductive paste of the second area in order to make one or more multi-layer conductive areas.

The first and second conductive pastes 20a, 20b are not necessarily in contact with each other; other layers can be interposed between said pastes 20a, 20b.

Clearly, also other conductive layers can be laid, depending on the type of structure to be made.

Preferably, at least one conductive area has a layer 12 of material varying its electric features on varying of the external mechanical stresses.

In particular, this layer of material can be interposed between two layers of conductive material (the conductive pastes 20a, 20b, for example) belonging to the same conductive area.

In this manner, this conductive area can be used to make sensors detecting deformations, forces (a weight variation, for example), etc.

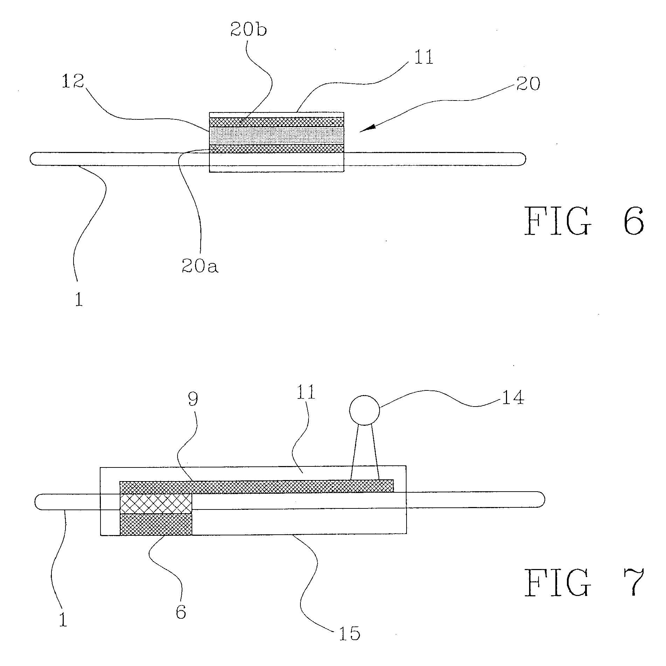

Shown in Fig. 6 is an example of a multi-layer sensor 20 in which the resistivity value is varying as a function of the pressure applied along the perpendicular line of the sensor itself.

If, for instance, layer 12 of soft, spongy, conductive material is inserted between two conductive layers 20a and 20b, the conductivity of same will be varied depending on compression. This layer can be laid by means of the previously described process using the suitable resin that, as known in the art, will generate a spongy layer, and the conductive material, graphite for example, for making the paste.

It is to be noted that the above mentioned insulating layer interposed between the conductive area and the substrate is particularly useful for insulating the deformation-detecting sensor (or other physico-mechanical magnitude) from the textile or substrate on which it is engaged.

In order to send a signal from a conductive area to any external instrument or to send a signal from any external instrument to a conductive area, an interface 14 is provided which is made for example by crimping, riveting with clips or other suitable system.

In Fig. 7 the conductive area 6 is connected to other devices through a suitable conductor abutting on a metallic clip 14 placed on a conductive path 9, insulated both from the side in contact with the skin and towards the outside through the insulating layers 11, 15.

The sensors are therefore electric contact areas that can be connected with each other by conductors susceptible of many applications.

A primary application is obtained in the field of detecting body parameters. In this case, the textile can take the shape of a garment worn by a patient under clinical examination, in which the sensors are applied at suitable positions and are directly in contact with the skin of the patient, for an electrocardiographic detection for example.

The textiles being the object of the present invention enable articles of garment to be made that can be worn for many hours without particular inconvenience.

Another possible application can be, by way of example, that of acquiring also rough vital parameters (such as breath or heartbeat detection) and send them to a detector, also remote, that can be able to send alarms. Let us think of newborn babies to be monitored in the cradle to prevent respiratory arrests or of persons performing high-risk operations.

It is also possible to make articles of garment provided with sensors being the object of the present invention, disposed in such a manner as to carry out an electrical stimulation if connected to suitable generators for electrical stimulation. The advantage of an article thus made is to be able to carry out electrical stimulations without being obliged to manually position the individual sensors, which operation is tiresome and can give rise to errors.

Using the same technique, also flexible printed circuits for electronic applications can be made.

It is to be pointed out that the substrate in accordance with the invention can generally be used both for receiving signals from the human or animal body to which it is applied, and to transmit signals to said body, and also to vary signals coming from the body or addressed thereto.

The variation can for instance consist in a conversion of a physiological quantity - signals for pressure/deformation representative of the respiratory activity of the body under examination, for example - into an electrical quantity, i.e. the electric signal that is then transmitted from the substrate to an instrument for analysis associated therewith.

A similar speech can also apply to all other applications of the substrate, both in terms of reception and in terms of transmission, where there is a variation in the type of the treated signal.

The invention can be further clarified with the following examples.

### EXAMPLE 1

The paste hereinafter labelled as P25, has been obtained with 99 g of acrylic resin "NEO GLUE GLT", produced by "Lamberti S.p.A." - Gallarate (Italy), by mixing it with 1 g of "TUBASSIST FIX 104 W", produced by "CHT R. BEITLICH GmbH", Tubingen (Germany) and 25 g of "SIGRAFIL C" produced by "SGL TECHNICAL LTD", Leatherhead, United Kingdom. The components have been fully mixed to form the conductive paste P25. Other pastes have been obtained by varying the conductive-component concentration, according to the weights (g) reproduced in the table:

| ID | NEO PASTE GLT | TUBASSIST FIX 104 W | SIGRAFIL C | Resistance (ohm) |
|---|---|---|---|---|
| P0 | 99 | 1 | 0 | Non conductive |
| P15 | 99 | 1 | 15 | 67.5 |
| P25 | 99 | 1 | 25 | 51.5 |
| P35 | 99 | 1 | 35 | 33 |
| P50 | 99 | 1 | 50 | 21.5 |

A cotton textile was placed on a table for making silk-screen printings on cloth. A circular hole of a 4 cm diameter was formed in a plate of polypropylene having a thickness of 2 mm and the plate too was placed on the printing table over said cloth. The paste P25 was spread on the cloth by use of a doctor blade in the region defined by the die hole. The die was separated from the textile and the latter was introduced into a drying oven at 130°C for three minutes and three times. Other pastes prepared in the same manner as P25 but with a composition as defined in the table were applied onto the textile following the described process. Then the resistance of the conductive regions was measured and the results are reproduced in the table; these values were obtained by making an average of the measurements carried out in different conductive areas.

### EXAMPLE 2

Six conductive regions were printed on the same type of cotton textile as in Example 1 and these regions were such localised as to enable manufacture of a T-shirt in which the conductive regions could be placed at the trunk of the individual designed to wear it. The paste P35 was used with a six-hole die to create the conductive regions. An insulated conductor cable having a stripped end was inserted in the selected places. The paste was spread with a doctor blade and the cables were incorporated into the conductive region. Polymerisation of the paste was accelerated by use of hot air, following the procedure described in Example 1. A T-shirt was made with the textile prepared as described, The T-shirt was used to measure an electrocardiogram with very satisfactory results.

## Claims

1. A process for making a flexible substrate provided with one or more electrically conductive areas, **characterised in that** it comprises the following steps:
- providing a flexible substrate (1) made of a sheet material;
- providing forming means (4) for at least one first electrically conductive area, said means (4) having a profile (4a) delimiting an area defined on said substrate (1), and a thickness defined along a direction substantially orthogonal to said substrate (1) ;
- applying an electrically conductive paste onto said flexible substrate (1) by means of said forming means (4), thereby obtaining a first electrically conductive area (6, 6a) having the shape delimited by the area and the thickness defined by said profile (4a);
- causing solidification of the conductive paste of said first conductive area (6, 6a);
- causing mutual engagement between the conductive paste of said first conductive area (6, 6a) and said substrate (1).

2. A process as claimed in claim 1, **characterised in that** it further comprises a step of providing an electrically non conductive layer (7) between said substrate (1) and first conductive area (6, 6a).

3. A process as claimed in claim 1 or 2, **characterised in that** it further comprises a step of engaging a conductive cable (3) with said substrate (1), said first conductive area (6, 6a) being obtained at an end (2) of said conductive cable (3) and being in electrical contact with said end (2).

4. A process as claimed in anyone of the preceding claims, **characterised in that** said first conductive area (6a) is formed on a first surface (1a) of said substrate (1), said process further comprising the following steps:
- providing forming means for a second electrically conductive area, said means having a profile delimiting an area defined on said flexible substrate (1), and a thickness defined along a direction substantially orthogonal to said flexible substrate (1);
- applying through said forming means, an electrically conductive paste onto a second surface (1b) of said flexible substrate (1) opposite to said first surface (1a), thereby obtaining a second electrically conductive area (6b) having a shape delimited by the area and thickness defined by said profile, said first and second conductive areas (6a, 6b) overlapping one another at least partly along an axis (Y) orthogonal to said substrate (1);
- causing solidification of the conductive paste of said second conductive area (6b);
- causing mutual engagement between the conductive paste of said second conductive area (6b) and said substrate (1).

5. A process as claimed in anyone of the preceding claims, **characterised in that** it further comprises a microperforation step carried out on at least part of a predetermined region (16) of said substrate (1).

6. A process as claimed in claims 4 and 5, **characterised in that** said predetermined region (16) is defined by the overlapped region between said first and second conductive areas (6a, 6b).

7. A process as claimed in anyone of the preceding claims, **characterised in that** it further comprises a step of providing an insulating layer (11) on said first area (6, 6a) and/or said second area (6b) on the opposite side relative to said flexible substrate (1).

8. A process as claimed in anyone of the preceding claims, **characterised in that** it further comprises a step of providing in said first conductive area (6, 6a) and/or said second conductive area (6b), a layer of material varying its electrical features on varying of the external mechanical stresses.

9. A process as claimed in anyone of the preceding claims, **characterised in that** it comprises a step of providing a second conductive paste (20a) positioned on the conductive paste (20a) of said first area (6, 6a) and/or on the conductive paste (20a) of said second area (6b) for making one or more multi-layer conductive areas (20).

10. A flexible substrate, in particular a textile, provided with one or more conductive areas (6, 6a, 6b) and made by the process according to anyone of the preceding claims.

11. Use of the flexible substrate (1) according to claim 10 for receiving and/or varying and/or transmitting electric signals from/to a human or animal body, said flexible substrate (1) being brought into contact with said human or animal body, in particular for measurement of clinical and/or physiological parameters and/or parameters representative of conditions or mechanical variations of said human or animal body.

12. A device for measurements of the clinical and/or physiological type and/or of parameters representative of conditions or mechanical variations of said human or animal body, to be carried out through contact with a human or animal body, **characterised in that** it comprises a flexible substrate (1) provided with one or more conductive areas (6, 6a, 6b) according to claim 10.

13. A sensor for detection of mechanical quantities, in particular deformations and/or pressures, **characterised in that** it comprises a flexible substrate (1) provided with one or more conductive areas (6, 6a, 6b) according to claim 10.
